# EUROPEAN PATENT SPECIFICATION

(11) **EP 3 247 286 B1**
(45) Date of publication and mention of the grant of the patent: **06.05.2020**
(21) Application number: 15878354.8
(22) Date of filing: 20.01.2015
(51) Int. Cl.: A61B 17/128, A61B 17/03

(54) **SURGICAL CLIP APPLIER WITH MULTIPLE CLIP FEEDING MECHANISM**
APPLIKATOR FUER CHIRURGISCHE KLAMMERN MIT MECHANISMUS ZUR ZUFÜHRUNG MEHRERER KLAMMERN
APPLICATEUR D'AGRAFES CHIRURGICALES AVEC MÉCANISME D'ALIMENTATION D'AGRAFES MULTIPLE

(43) Date of publication of application: 29.11.2017
(73) Proprietor: Covidien LP, Mansfield, MA 02048 (US)
(72) Inventor: HU, Encheng, Shanghai 201199 (CN); XU, Shunhong, Shanghai 201199 (CN); TAN, Yuandong, Jiangsu 211100 (CN); CHEN, Lin, Shanghai 201101 (CN); CAI, Longsheng, Shanghai 201112 (CN)
(74) Representative: Maschio & Soames IP Ltd
(86) International application number: PCT/CN2015/071089
(87) International publication number: WO 2016/115676

(56) References cited:
- EP-A1- 2 609 877
- WO-A1-00/42922
- CN-A- 103 181 810
- CN-A- 103 976 771
- CN-A- 104 027 141
- US-A- 4 500 024
- US-A- 5 030 226
- US-A1- 2012 048 759
- US-A1- 2013 131 697

## Description

### BACKGROUND

### 1. Technical Field

The present disclosure relates to surgical clip appliers. More particularly, the present disclosure relates to reposable surgical clip appliers having a disposable clip cartridge assembly for use in open or endoscopic surgical procedures.

### 2. Discussion of Related Art

Surgical clip appliers are known in the art and have increased in popularity among surgeons by offering an alternative to conventional suturing of body tissues and vessels. Typical instruments are disclosed in U.S. Pat. No. 5,030,226 to Green et al. and U.S. Pat. No. 5,431,668 to Burbank, III et al. These instruments generally provide a plurality of clips which are stored in the instrument and which are fed sequentially to the jaw mechanism at the distal end of the instrument upon opening and closing of the handles at the proximal end of the instrument. As the handles are closed, the jaws close to deform a clippositioned between the jaw members, and as the jaws are opened to release the deformed clip, a new clip is fed from the series of clips to a position between the jaws. This process is repeated until all the clips in the series of clips have been used.

A cartridge with a plurality of C-shaped clips, a clip tray and first and second transport elements with holding tongues, is shown in US-A-2012/0048759.

During open, endoscopic, or laparoscopic procedures, it may be desirable and/or necessary to use different size surgical clips depending on the underlying tissue or vessels to be ligated. In order to reduce overall costs of a surgical clip applier, it is desirable for a single surgical clip applier to be loadable with and capable of firing different size surgical clips as needed.

Accordingly, a need exists for surgical clip appliers that include disposable clip cartridge assemblies and reusable handle assemblies.

### SUMMARY

The present invention is defined in independent claim 1 and certain optional features of the present invention are defined in the dependent claims. The present disclosure relates to reposable endoscopic surgical clip appliers.

According to an aspect of the present disclosure, a clip cartridge assembly is provided and includes a clip tray, a cartridge clip pusher bar, and a plurality of surgical clips interposed between the clip tray and the cartridge clip pusher bar.

The clip tray is configured to be selectively loadable in a channel assembly of a surgical clip applier and includes a first plurality of distally oriented, deflectable, resilient fingers projecting from a base wall thereof,wherein a surgical clip is disposed distally of each resilient finger of the clip tray.

The cartridge clip pusher bar is disposed adjacent the clip tray and is slidable relative thereto. The cartridge clip pusher bar includes a second plurality of distally oriented, deflectable, resilient fingers projecting from a base wall thereof which each terminate in a distal shoulder.

The cartridge clip pusher bar may include a stem disposed at and projection from a proximal end of the cartridge clip pusher bar. The stem may be configured to slidably extend through a channel defined in an inner surface of the clip tray. The cartridge clip pusher bar may be urged distally relative to the clip tray.

In use, distal movement of the cartridge clip pusher bar causes each distal shoulder of the cartridge clip pusher bar to contact a backspan of a respective surgical clip, thereby distally advancing all of the surgical clips simultaneously.

Following distal movement of the cartridge clip pusher bar, upon a proximal movement of the cartridge clip pusher bar, each of the second plurality of distally oriented, deflectable, resilient fingers contacts the backspan of a respective remaining one of the surgical clip to proximally move all of the remaining surgical clips until the backspans of all of the remaining surgical clips contacts a respective finger of the first plurality of distally oriented, deflectable, resilient fingers of the clip tray to block any further proximal movement of the remaining surgical clips.

The clip tray may include a spaced apart pair of distally oriented, deflectable, resilient fingers projecting from a distal end of the base wall thereof configured to contact a distal surface of a backspan of a surgical clip being distally urged by the cartridge clip pusher bar, thereby inhibiting any further distal movement of the surgical clip.

The distal-most finger of the first plurality of distally oriented, deflectable, resilient fingers may have a rectangular configuration.

Each remaining finger of the first plurality of distally oriented, deflectable, resilient fingers may include a V-shaped notch formed in a distal end thereof.

A distal end of the cartridge clip pusher bar may include a pusher disposed thereon defining a pair of spaced apart prongs configured to engage a backspan of a surgical clip.

The clip cartridge assembly may include a channel cover configured to selectively engage the clip tray. The channel cover may be configured to provide a biasing force on the cartridge clip pusher bar such that the cartridge clip pusher bar is maintained in a position adjacent to the clip tray.

According to another aspect of the present disclosure, a surgical clip applier is provided and includes a housing, at least one handle pivotably connected to the housing, a channel assembly extending distally from the housing, a clip cartridge assembly selectively loadable in the channel assembly being in mechanical communication with the at least one handle, and a pair of jaws supported on a distal end of the channel assembly configured to receive a single, unformed surgical clip therein.

The clip cartridge assembly includes a clip tray, a cartridge clip pusher bar, and a plurality of surgical clips interposed between the clip tray and the cartridge clip pusher bar.

The clip tray is configured to be selectively loadable in a channel assembly of the surgical clip applier, and includes a first plurality of distally oriented, deflectable, resilient fingers projecting from a base wall thereof, wherein a surgical clip is disposed distally of each resilient finger of the clip tray.

The cartridge clip pusher bar is disposed adjacent the clip tray and is slidable relative thereto. The cartridge clip pusher bar includes a second plurality of distally oriented, deflectable, resilient fingers projecting from a base wall thereof and each terminates in a distal shoulder. A proximal end of the cartridge clip pusher bar is configured for selective engagement with a drive assembly of the surgical clip applier, such that the cartridge clip pusher bar is in mechanical communication with the at least one handle of the surgical clip applier.

The cartridge clip pusher bar may include a stem disposed at and projection from a proximal end of the cartridge clip pusher bar. The stem may be configured to slidably extend through a channel defined in an inner surface of the clip tray and selectively engage a drive assembly of the surgical clip applier, such that actuation of the at least one handle of the surgical clip applier causes the drive assembly of the surgical clip applier to act on the stem of the cartridge clip pusher bar, wherein the cartridge clip pusher bar is urged distally relative to the clip tray.

In use, distal movement of the cartridge clip pusher bar causes each distal shoulder of the cartridge clip pusher bar to contact a backspan of a respective surgical clip, thereby distally advancing all of the surgical clips simultaneously.

Following distal movement of the cartridge clip pusher bar, upon a proximal movement of the cartridge clip pusher bar, each of the second plurality of distally oriented, deflectable, resilient fingers contacts the backspan of a respective remaining one of the surgical clip to proximally move all of the remaining surgical clips until the backspans of all of the remaining surgical clips contacts a respective finger of the first plurality of distally oriented, deflectable, resilient fingers of the clip tray to block any further proximal movement of the remaining surgical clips.

The clip tray may include a spaced apart pair of distally oriented, deflectable, resilient fingers projecting from a distal end of the base wall thereof configured to contact a distal surface of a backspan of a surgical clip being distally urged by the cartridge clip pusher bar, thereby inhibiting any further distal movement of the surgical clip.

The distal-most finger of the first plurality of distally oriented, deflectable, resilient fingers may have a rectangular configuration.

Each remaining finger of the first plurality of distally oriented, deflectable, resilient fingers may include a V-shaped notch formed in a distal end thereof.

A distal end of the cartridge clip pusher bar may include a pusher disposed thereon defining a pair of spaced apart prongs configured to engage a backspan of a surgical clip.

The reposable surgical clip applier may include a channel cover configured to selectively engage the clip tray. The channel cover may be configured to provide a biasing force on the cartridge clip pusher bar such that the cartridge clip pusher bar is maintained in a position adjacent to the clip tray.

The surgical clip applier may include an endoscopic assembly selectively connectable to the housing, wherein the channel assembly is disposed within the endoscopic assembly. A distal end of the endoscopic assembly may include a pair of jaws supported thereon.

### BRIEF DESCRIPTION OF THE DRAWINGS

The present clip applier will be more fully appreciated as the same becomes better understood from the following detailed description when considered in connection with the following drawings, in which:
FIG. 1 is a top, perspective view, of a surgical clip applier according to an embodiment of the present disclosure;
FIG. 2 is a top, perspective view, of a clip cartridge assembly of the surgical clip applier of FIG. 1;
FIG. 3 is a bottom, perspective view, of the clip cartridge assembly of FIG. 2;
FIG. 4 is an enlarged view of the indicated area of detail of FIG. 2;
FIG. 5 is an enlarged view of the indicated area of detail of FIG. 3;
FIG. 6 is a top, perspective view, of the clip cartridge assembly of FIG. 2, with parts separated;
FIG. 6A is a perspective view, with parts separated, of the surgical clip applier of FIG. 1;
FIG. 6B is a schematic perspective view of a surgical clip applier according to an alternate embodiment of the present disclosure;
FIG. 7 is an enlarged perspective view of the clip cartridge assembly of FIG. 2, showing a cartridge clip pusher bar separated from a clip tray;
FIG. 8 is a cross-sectional view of the shaft assembly as viewed along 8-8 of FIG. 2;
FIG. 9 is an enlarged view of the indicated area of detail of FIG. 8;
FIG. 10 is a side cross-sectional view of the surgical clip applier of FIG. 9 during an initial squeezing of the handles;
FIG. 11 is another side, cross-sectional view of the surgical clip applier of FIG. 9 during a continued squeezing of the handles;
FIG. 12 is another side, cross-sectional view of the surgical clip applier of FIG. 9 during a further continued squeezing of the handles;
FIG. 13 is another side, cross-sectional view of the surgical clip applier of FIG. 9, during an initial un-squeezing of the handles;
FIG. 14 is another side, cross-sectional view of the surgical clip applier of FIG. 9, during a continued un-squeezing of the handles; and
FIG. 15 is another side, cross-sectional view of the surgical clip applier of FIG. 9, during a further un-squeezing of the handles.

### DETAILED DESCRIPTION OF EMBODIMENTS

Embodiments of reposable surgical clip appliers, in accordance with the present disclosure, will now be described in detail with reference to the drawing figures wherein like reference numerals identify similar or identical structural elements. As shown in the drawings and described throughout the following description, as is traditional when referring to relative positioning on a surgical instrument, the term "proximal" refers to the end of the apparatus which is closer to the user and the term "distal" refers to the end of the apparatus which is further away from the user.

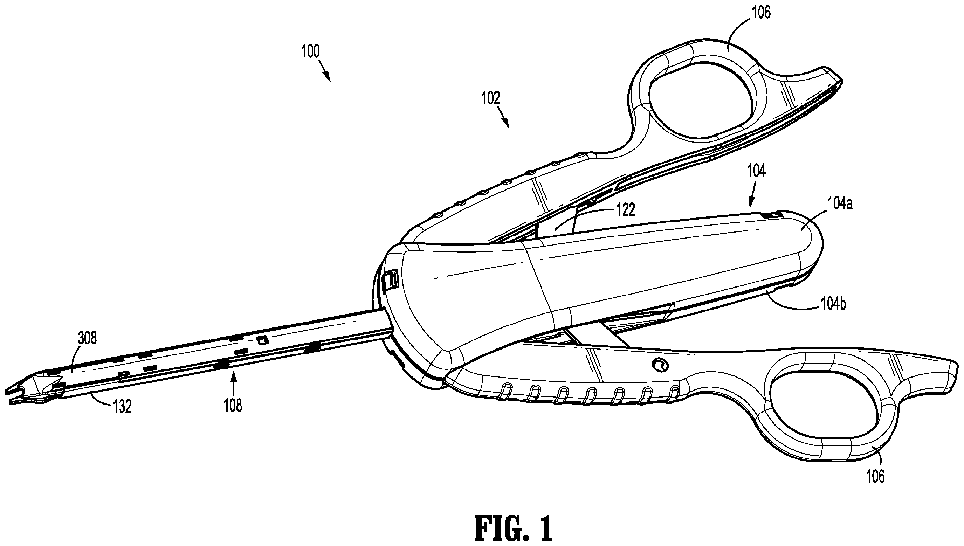

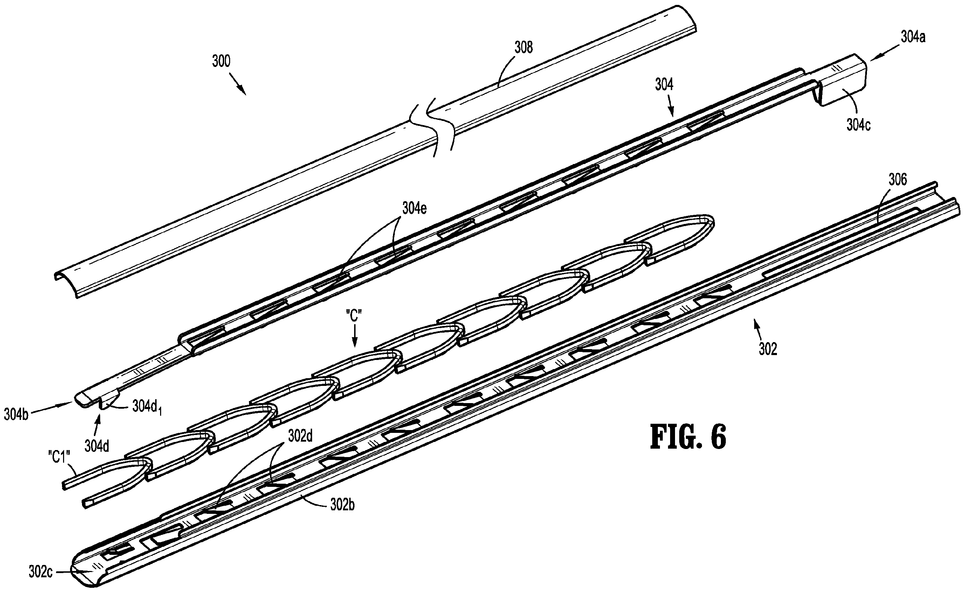

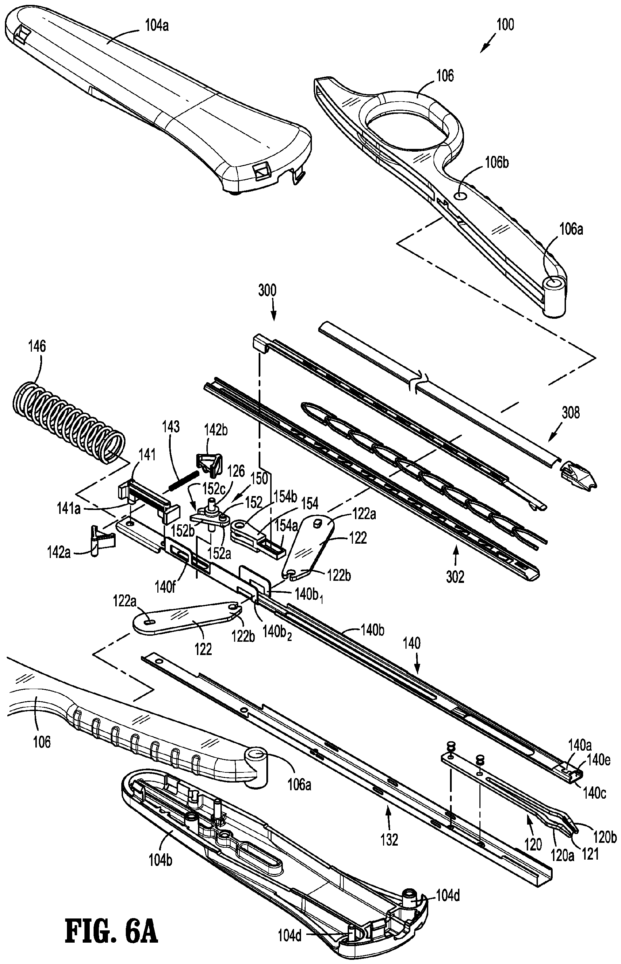

Referring now to FIG. 1, a surgical clip applier in accordance with an embodiment of the present disclosure is generally designated as 100. It is contemplated that any of the components of surgical clip applier 100 may be formed from any suitable biocompatible material such as stainless steel, titanium, or the like. Surgical clip applier 100 generally includes a handle assembly 102 including a housing 104 having upper housing half 104a and lower housing half 104b. Handle assembly 102 further includes a pair of handles 106pivotably secured to housing 104 and extending outwardly therefrom. A channel assembly 108 is fixedly secured to housing 104 and extends outwardly therefrom, terminating in and supporting a jaw assembly 110. As shown in FIG. 6A, housing halves 104a and 104b fit together by snap fit engagement with one another. It is further contemplated that housing halves 104a and 104b may be joined through one or more screws, rivets, or the like, or through the use of glues or other adhesives.

As shown in FIG. 6A, handles 106 are pivotably secured to housing 104 by handle pivot posts 104d extending from lower housing half 104b and into respective distal apertures 106a formed in handles 106. Handle assembly 102 includes a link member 122 pivotally connected at a first end 122a thereof to each handle 106 at a pivot point 106b formed in a respective handle 106. A second end 122b of each link member 122 is pivotally connected to a respective pivot point 140b₁, 140b₂ defined in side walls 140b of drive channel 140.

Referring again to FIG. 1, channel assembly 108 includes an outer or lower channel 132 having a proximal end retained in housing 104, between upper and lower housing halves 104a, 104b.

Referring now to FIG. 6A, clip applier 100 includes a drive channel 140 reciprocally supported in and extending between housing 104 of handle assembly 102 and channel assembly 108. A proximal end of drive channel 140 is supported between upper and lower housing halves 104a, 104b of housing 104 and a distal end of drive channel 140 is supported by outer channel 132 of channel assembly 108.

A distal end of drive channel 140 is a substantially box-shaped tube including a pair of spaced apart side walls 140b interconnecting a top wall 140a and a bottom wall 140c. The distal-most end of drive channel 140 includes a tongue 140e disposed between side walls 140b and extending from and between top wall 140a and bottom wall 140c.

With continued reference to FIG. 6A, clip applier 100 further includes a biasing member 146, in the form of a compression spring, disposed within housing 104 so as to operatively surround an intermediate portion of drive channel 140 and to abut against an inner distal surface of housing 104. Biasing member 146 functions to generally maintain drive channel 140 in a retracted or proximal-most position. In particular, biasing member 146 functions to retract or facilitate retraction of drive channel 140 following formation of a surgical clip "C" positioned between jaws 120, and following release of handles 106.

A proximal end of drive channel 140 supports a ratchet rack member 141 that is secured thereto, wherein ratchet rack member 141 is movable together with drive channel 140. Ratchet rack member 141 defines a first set of teeth 141a configured and adapted to engage with a first ratchet pawl 142a, and second set of teeth 141b (not shown) disposed on an opposite side thereof configured and adapted to engage a second ratchet pawl 142b. First and second ratchet pawls 142a, 142b are each pivotally supported in housing 104, with first and second ratchet pawls 142a, 142b being disposed on opposed sides of ratchet rack member 141. Rack member 141 and pawls 142a, 142b define a ratchet mechanism 144. In use, as drive channel 140 is moved axially, rack member 141 is also moved axially therewith. The series of rack teeth 141a, 141b have a length which allows respective pawls 142a, 142b to reverse and advance back over rack member 141 when rack member 141 changes between proximal and distal movement after drive channel 140 reaches a proximal-most or distal-most position.

Each pawl 142a, 142b is pivotally supported in upper housing half 104a and lower housing half 104b at a location wherein each pawl 142a, 142b is in substantial operative engagement with respective rack teeth 141a, 141b of rack member 141. Pawls 142a, 142b are engageable with rack member 141 to restrict longitudinal movement of rack member 141 and, in turn, drive channel 140.

Ratchet mechanism 144 includes a snap-over spring 143, in the form of a coil spring, interposed between pawls 142a, 142b and extending through and across rack member 141. Snap-over spring 143 functions to maintain the teeth of pawls 142a, 142b in engagement with the respective rack teeth 141a, 141b of rack member 141 as rack member 141 is axially translated.

In operation, when drive channel 140 is moved to the distal-most position and rack teeth 141a, 141b of rack member 141 have cleared pawls 142a, 142b, a surface of rack member 141 engages snap-over spring 143 to reverse the direction of buckling thereof, and thus reverse or change the orientation of pawls 142a, 142b to thereby permit proximal movement of drive channel 140. Additionally, when drive channel 140 is moved to the proximal-most position and rack teeth 141a, 141b of rack member 141 have once again cleared pawls 142a, 142b, another surface of rack member 141 engages snap-over spring 143 to again reverse the direction of buckling thereof, and thus again reverse or change the orientation of pawls 142a, 142b to thereby permit distal movement of drive channel 140.

Clip applier 100 is provided with audible/tactile indication or feedback when pawls 142a, 142b are flipped at either end of the stroke of drive channel 140. In particular, when pawls 142a, 142b flip, pawls 142a, 142b are accelerated by snap-over spring 143 and slap against the walls of housing 104, thereby providing the user feedback that the end of a stroke has been reached. For a detailed discussion of the construction and operation of an exemplary ratchet mechanism, reference may be made to U.S. Patent Application Publication No. 014/0194903.

As illustrated in FIG. 6A, jaw assembly 110 of clip applier 100 includes a pair of jaws 120 mounted on or at a distal end of channel assembly 108 and actuatable by handles 106 of handle assembly 102. The pair of jaws 120 is formed of a suitable biocompatible material such as, for example, stainless steel or titanium. The pair of jaws 120 includes a first jaw member 121a, and a second jaw member 121b, which define a channel 121 there between for receipt of a surgical clip "Cl" therein. For a detailed discussion of the construction and operation of exemplary jaws, reference may again be made to U.S. Patent Application Publication No. 2014/0194903, as referenced hereinabove.

Continuing with FIG. 6A, clip applier 100 further includes linkage mechanism 150 having a distal, driven link arm 154 having a first, distal end 154a selectively connectable to proximal end 160b of pusher bar 304 of clip cartridge assembly 300, as described in further detail below. Linkage mechanism 150 further includes a proximal, driver link arm 152 having a first, distal end 152a pivotally connected to a second, proximal end 154b of driven link arm 154, and a second, proximal end 152b defining an arcuate slot 152c configured to slidably receive a tab 140f of drive channel 140. Arcuate slot 152c is open ended and is only engaged by tab 140f of drive channel 140 during select portions of a distal and proximal translation thereof, as will be described in greater detail below. Driver link arm 152 is pivotally connected to outer channel 132 via a pin 126, such that driver link arm 152 is axially fixed with respect to outer channel 132.

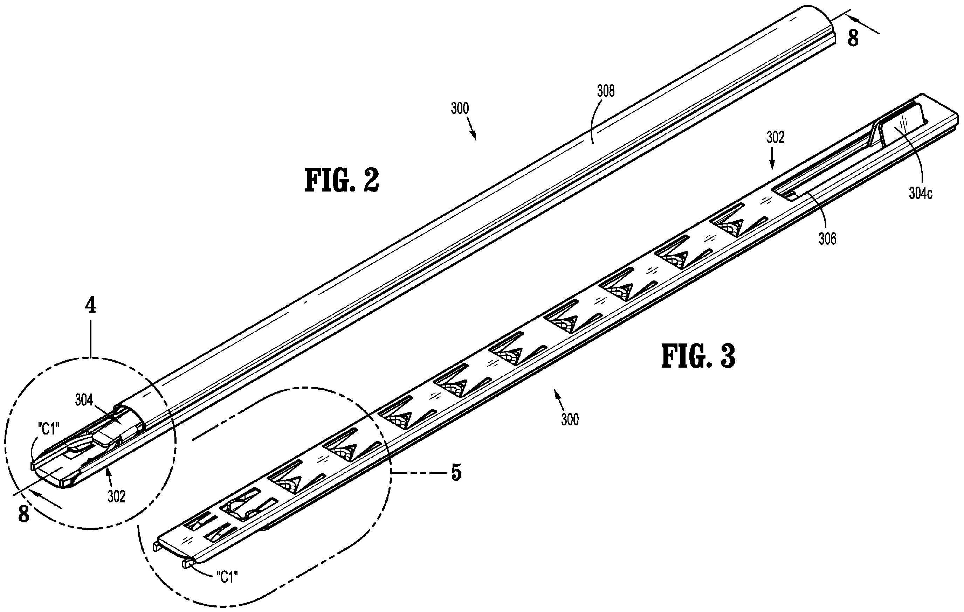

Turning now to FIGS. 2-7, clip cartridge assembly 300 of surgical clip applier 100 is shown. Clip cartridge assembly 300 is configured to be selectively loadable into outer channel 132 of channel assembly 108, and to be actuated by handle assembly 102 to fire and form the surgical clips loaded therein onto underlying tissue and/or vessels. Each clip cartridge assembly 300 may be loaded with a particularly sized set of surgical clips (e.g, relatively small surgical clips, relatively medium surgical clips, or relatively large surgical clips).

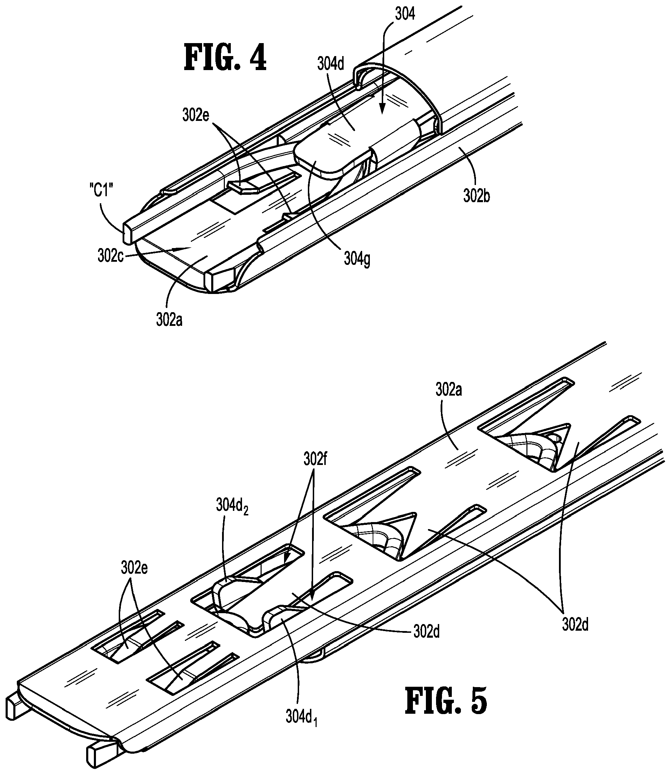

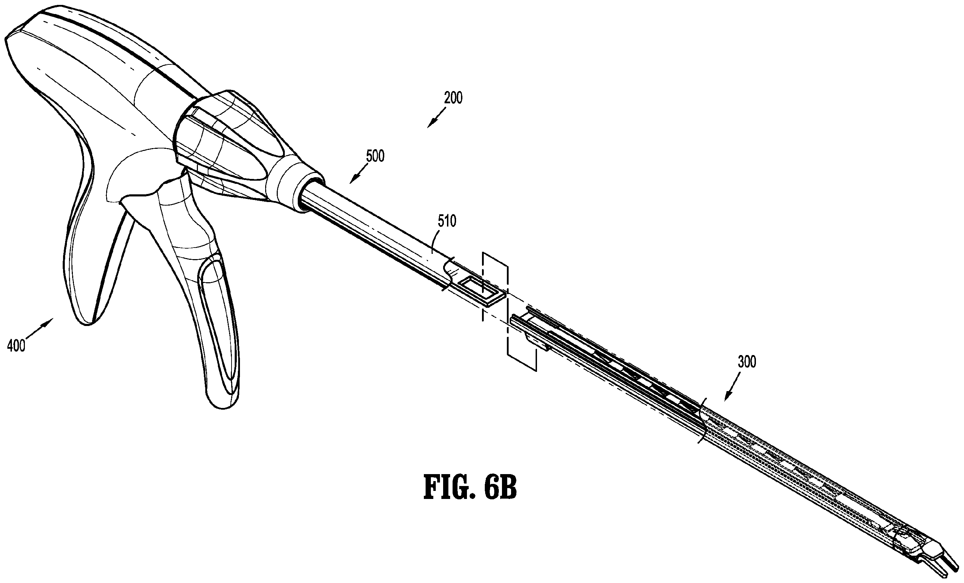

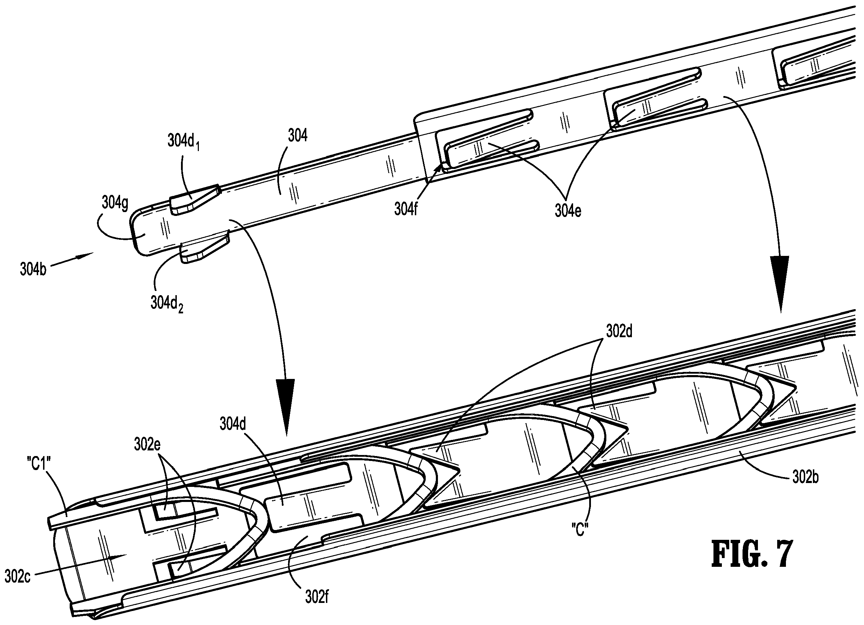

Clip cartridge assembly 300 includes a clip tray 302 including base wall 302a and a pair of spaced apart side walls or rails 302b supported on base wall 302a. Base wall 302a and side walls 302b define a clip channel 302c. Clip tray 302 includes a linear array of distally extending resilient, deflectable fingers 302d projecting up from base wall 302a and into clip channel 302c at a location between side walls 302b. The distal-most deflectable finger 302d forms a single, rectangular finger configured to engage a backspan or crown of the distal-most clip "Cl" and nest between the spaced apart prongs 304d₁, 304d₂ (FIG. 5) of cartridge clip pusher bar 304, as discussed below. A pair of channels 302f are disposed on either side of the distal-most finger 302d and are configured to selectively receive each of the spaced apart prongs 304d₁, 304d₂ (FIG. 5) of clip pusher bar 304 when all of the surgical clips "C" have been formed, as will be discussed in further detail below. Each of the remaining proximal deflectable fingers 302d define a V-shaped configuration adapted to engage opposing sides of a backspan of each remaining clip of the stack of surgical clips "C" as illustrated in FIG. 7. A slot or channel 306 (FIGS. 3 and 6) is defined within the proximal end of base wall 302a such that the coupling stem 304c of cartridge clip pusher bar 304 may be slidably disposed therein, as discussed in further detail below.

As shown in FIGS. 4 and 5, clip tray 302 further includes a pair of spaced apart, distally extending resilient deflectable prongs 302e projecting up from the proximal end of base wall 302a and into clip channel 302c at a location between side walls 302b. Each of the prongs 302e is spaced apart such that they are in slidable engagement with an inner portion of each leg of the distal-most clip "C1". Prongs 302e are dimensioned to engage the interior of a backspan of the clip "C", such that prongs 302e prevent the clip, proximal to the distal-most clip "Cl", from advancing distally past prongs 302e when the distal-most clip "Cl" is being loaded into the pair of jaws 120. The biasing force provided by the prongs 302e is overcome when the distal-most clip "C" is urged distally by the cartridge clip pusher bar 304, and once the distal-most clip "Cl" has been loaded into the pair of jaws 120, each of the prongs 302e returns to their initial position to prevent the next clip "C" from advancing any further.

Clip cartridge assembly 300 includes a cartridge clip pusher bar 304slidably disposed adjacent clip tray 302. Cartridge clip pusher bar 304 includes a proximal end 304adefining a coupling stem, head, or boss 304c configured to slidably protrude through channel 306 of clip tray 302 and selectively connect with distal end 154a of linkage mechanism 150. Cartridge clip pusher bar 304 further includes a distal end portion 304b defining a pusher 304d configured to engage a distal-most clip "Cl" of a stack of surgical clips "C" for loading the distal-most surgical clip "Cl" into the pair of jaws 120 of clip applier 100. Pusher 304d includes a pair of spaced apart prongs 304d₁, 304d₂ (FIG. 7) configured to engage a backspan of the distal-most surgical clip "Cl", as illustrated in FIGS. 4-5.

As illustrated in FIG. 7, cartridge clip pusher bar 304 further includes a linear array of distally extending resilient, deflectable fingers 304e, with each finger 304e defining a rectangular profile including a distal shoulder 304f disposed on a distal end thereof. In use, when cartridge clip pusher bar 304 overlays or is adjacent to clip tray 302, each of the deflectable fingers 302d of clip tray 302 is disposed axially between each deflectable finger 304e of cartridge clip pusher bar 304, such that each of deflectable fingers 302d, 304e are arranged in a tandem configuration when the clip pusher bar 304 is in a proximal position relative to clip tray 302.

In use, the V-shaped configuration of the proximal deflectable fingers 302d of clip tray 302 permits each proximal deflectable finger 304e of the cartridge clip pusher bar 304 to nest within the V-shape of the proximal deflectable fingers 302d such that distal shoulder 304f of deflectable finger 304e engages a backspan of each of the remaining surgical clips of the stack of surgical clips "C" as the cartridge clip pusher bar 304 is driven distally.

Clip cartridge assembly 300 includes a stack of surgical clips "C" interposed between clip tray 302 and cartridge clip pusher bar 304. The stack of surgical clips "C" is supported on or loaded in clip tray 302 such that a crown or backspan of each surgical clip "C" is disposed distal of a respective deflectable finger 302d of clip tray 302. Further, when cartridge clip pusher bar 304 is in a proximal position relative to clip tray 302, each deflectable finger 304e is also disposed proximal of the crown or backspan of a respective clip of the stack of surgical clips "C" as discussed above.

Clip cartridge assembly 300 may be loaded with 10 surgical clips "C", or, in embodiments, clip cartridge assembly 300 may be loaded with any number of surgical clips C", provided clip cartridge assembly 300and channel assembly 108 are appropriately configured and dimensioned. Surgical clips "C" may be fabricated from materials know by those skilled in the art, including and not limited to stainless steel, titanium, or other metal alloys. In an embodiment it is contemplated that at least a final surgical clip of the stack of surgical clips "C" may be dyed a particular color to indicate to the user when a final surgical clip of clip cartridge assembly 300 is loaded into the pair of jaws 120.

Once all of the surgical clips "C" are loaded into the clip tray 302 and cartridge clip pusher bar 304 is placed adjacent thereto, channel cover 308, configured and adapted for connection and support on clip tray 302,may be snapped into engagement with rails 302b of clip tray 302 such that channel cover 308 is detachably secured thereto. Channel cover 308 is configured and adapted to provide a downward biasing force on cartridge clip pusher bar 304, thereby preventing cartridge clip pusher bar 304 from separating from clip tray 302 during operation.

Once fully assembled as shown in FIGS. 2 and 3, clip cartridge assembly 300 may be loaded into channel assembly 108. Clip cartridge assembly 300 is loaded into channel assembly 108 by initially placing coupling stem 304cof the cartridge clip pusher bar 304 into selective engagement with distal end 154a of linkage mechanism 150. Thereafter, the clip tray 302 is pushed into further engagement with the side walls of outer channel 132 of channel assembly 108 such that clip tray 302, and therefore, clip cartridge assembly 300, are detachably secured thereto. Clip tray 302 may be secured to outer channel 132 of channel assembly 108 by any suitable means, such as a detent, lips, or wings. In one exemplary embodiment, wings extending along the side edges of outer channel 132 of channel assembly 108 and extending towards one another snap over or otherwise engage the lateral sides of clip tray 302 such that clip tray 302, and therefore, clip cartridge assembly 300, are detachably secured thereto.

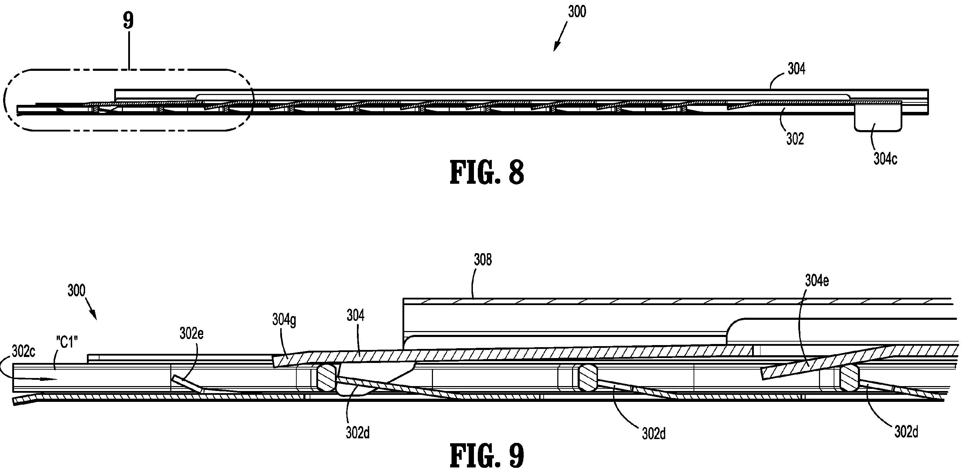

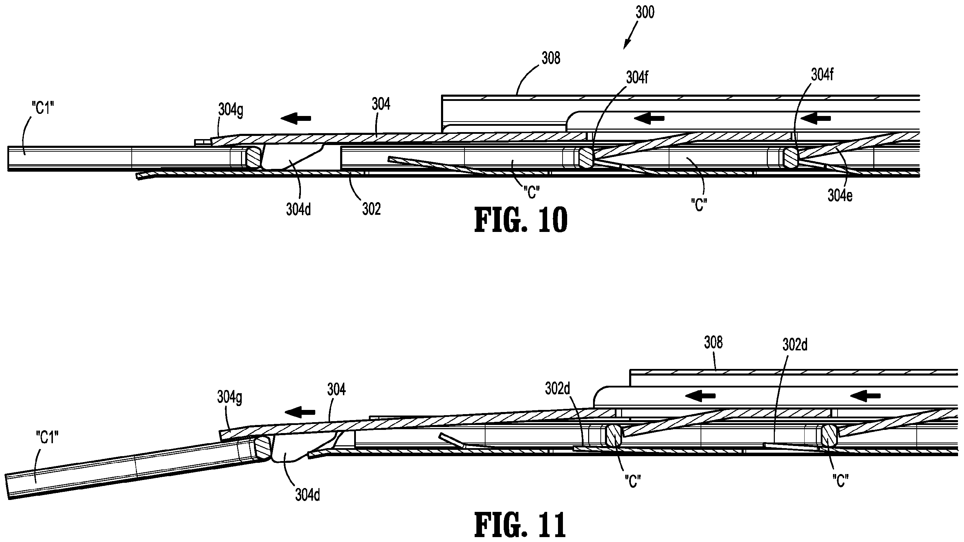

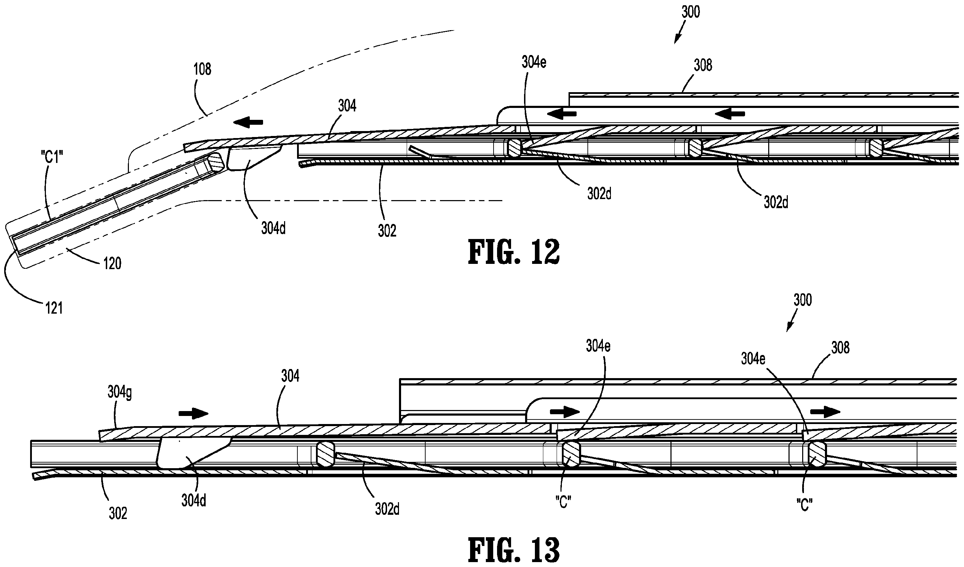

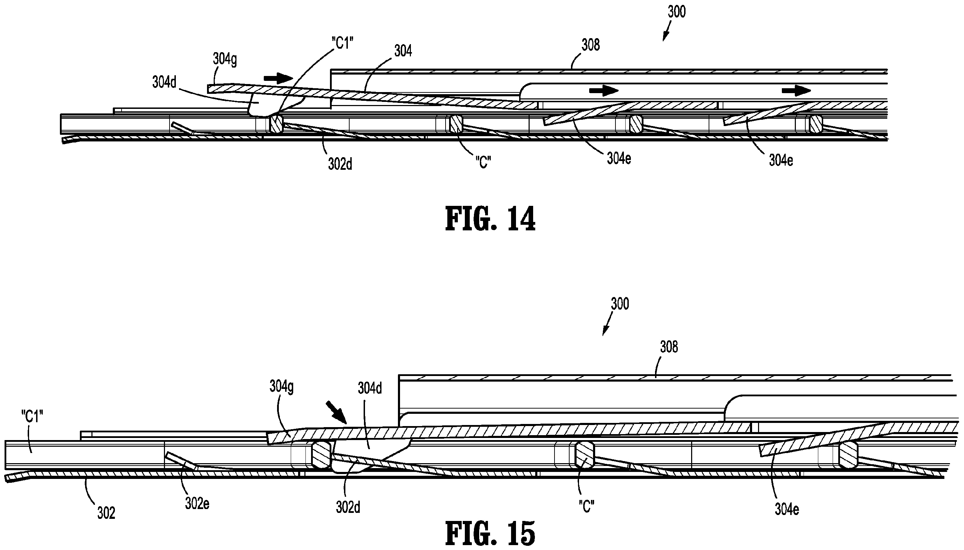

With continued reference to FIGS. 1-7, and with additional specific reference to FIGS. 8-15, an exemplary mode of operation of clip applier 100 is shown and described. As shown in FIGS. 8-15, clip applier 100 is illustrated with clip cartridge assembly 300 loaded in channel assembly 108 (as described above). The operation of clip applier 100 is substantially similar to that of the exemplary clip applier referenced hereinabove in U.S. Patent Application Publication No. 2014/0194903, and therefore, in the interest of brevity, only the differences therebetween will be discussed hereinbelow.

As the linkage mechanism 150 is urged distally by drive bar 140, the cartridge clip pusher bar 304 of clip cartridge assembly 300 is moved in a distal direction to load distal-most clip "Cl" of the stack of surgical clips "C" in a distal direction and into the pair of jaws 120. Specifically, the spaced apart prongs 304d₁, 304d₂ of pusher 304d of cartridge clip pusher bar 304 engages the backspan of distal-most clip "Cl" and pushes distal-most clip "Cl" out of clip cartridge assembly 300 and into channel 121 of the pair of jaws 120. As mentioned above, the distal movement of the distal-most clip "Cl" compresses each of the spaced apart prongs 302e of the clip tray 302, thereby permitting the distal-most clip "Cl" to be loaded within the pair of jaws 120, as described above. Once the distal-most clip "C1" is loaded into the pair of jaws 120, the prongs 302e return to their initial position and prevent the clips "C", proximal to the distal-most clip "Cl", from advancing further distally.

Additionally, and simultaneously with a distal movement of the distal-most clip "Cl", as cartridge clip pusher bar 304 of clip cartridge assembly 300 moves in a distal direction, distal shoulder 304f of finger 304eof cartridge clip pusher bar 304 abuts against a respective backspan of a respective surgical clip of the remaining surgical clips "C" to also urge the remaining surgical clips "C" in a distal direction, such that fingers 302d of the clip tray 302 are deflected, thereby permitting each of the remaining surgical clips "C" to advance distally to replace each respective surgical clip of the stack of surgical clips "C". Once distal advancement of each surgical clip "C" is complete, fingers 302d of clip tray 302 spring back or return to their original position, thereby preventing each surgical clip "C" from moving in a proximal direction. Cartridge clip pusher bar 304 distally advances the remaining surgical clips "C" until each remaining surgical clip "C" is advanced distally past a next adjacent resilient, deflectable finger 302d of clip tray 302.

After completely forming the distal-most clip "Cl" within the pair of jaws 120, the handles 106 are opened causing the drive channel 140 to retract proximally, thereby causing linkage mechanism 150 to impart a force acting in a proximal direction on coupling stem 304c, thereby drawing cartridge clip pusher bar 304 proximally over each of the remaining surgical clips of the clip stack "C". As cartridge clip pusher bar 304 is moved in a proximal direction, fingers 304e abut against a distal surface of the backspans of the remaining surgical clips "C" to also urge the remaining surgical clips "C" in a proximal direction. Cartridge clip pusher bar 304proximally retracts the remaining surgical clips "C" until each remaining surgical clip "C" is retracted into contact with a respective distal tip of a respective resilient, deflectable finger 302d of clip tray 302, which blocks or stops further proximal retraction of the remaining surgical clips "C".

As cartridge clip pusher bar 304 continues to be drawn proximally, concurrently, the spaced apart prongs 304d₁, 304d₂ of pusher 304d and fingers 304e of cartridge clip pusher bar 304 are deflected up and over each of the remaining surgical clips "C" until cartridge clip pusher bar 304 returns to a proximal-most position. When clip cartridge assembly 300 is loaded with at least one clip "C", and when cartridge clip pusher bar 304 is in a proximal-most position, a nose 304g (FIG. 7)disposed on a distal-most end of cartridge clip pusher bar 304 is biased towards, and is supported by, an upper surface of a backspan of the distal-most surgical clip "Cl", preventing each of the spaced apart prongs 304d₁, 304d₂ of pusher 304d from engaging the pair of channels 302f of clip tray 302.

The operations described above can be repeated, as required, until all of the surgical clips "C" have been formed.

With reference to FIG. 5, an exemplary mode of operation of a lockout safety mechanism is illustrated. Once the final clip of the stack of clips "C" is formed and cartridge clip pusher bar 304 is retracted proximally, the nose 304g is no longer supported by a clip "C". The bias of the distal end 304b of cartridge clip pusher bar 304 downward toward clip tray 302 causes each of the spaced apart prongs 304d₁, 304d₂ of pusher 304dto engage the pair of channels 302f of clip tray 302. Once the spaced apart prongs 304d₁, 304d₂ of pusher 304d are engaged with or disposed within the pair of channels 302f of clip tray 302, any distal movement of the cartridge clip pusher bar 304 is inhibited. Further, and as described in detail in U.S. Patent Application Publication No. 2014/0194903, referenced above, since the handles 106 cannot be squeezed any further, and due to the engagement of pawls 142a, 142b with rack member 141, handles 106 cannot be reopened to any unsqueezed position, thereby locking the cartridge clip pusher bar 304 in place.

In use, surgical clip applier 100, as mentioned above, is capable of loading different surgical clip cartridge assemblies 300 in channel assembly 108. Specifically, channel assembly 108 may be loaded with a surgical clip cartridge assembly 300 that is loaded with a stack of surgical clips "C" having a first size, or channel assembly 108 may be loaded with a surgical clip cartridge assembly 300 that is loaded with a stack of surgical clips "C" having a second size different than the first size.

In this manner, the user or surgeon may load a surgical clip cartridge assembly 300, loaded with a particular size of surgical clips, depending on the particular surgical procedure to be performed. Additionally, during a surgical procedure, if the need arises to use a different sized surgical clip, the user or surgeon may eject or unload the surgical clip cartridge assembly 300 that is loaded in channel assembly 108and then load a new surgical clip cartridge assembly 300 (having a different sized stack of surgical clips loaded therein as compared to the unloaded surgical clip cartridge assembly 300) into channel assembly 108.

As illustrated in FIG. 6B, it is contemplated that surgical clip cartridge assembly 300 may be mated to a surgical clip applier 200 configured for use in endoscopic surgical procedures. Surgical clip applier 200 generally includes a handle assembly 400, and endoscopic assembly 500 including a shaft assembly 510 selectively connectable to and extendable distally from handle assembly 400, wherein the surgical clip cartridge assembly 300 is selectively loadable into shaft assembly 510 of endoscopic assembly 500. Assembly and operation of clip cartridge assembly 300 is identical to that which is described hereinabove, with the exception of being selectively attached to surgical clip applier 200. For a detailed discussion of exemplary endoscopic surgical clip appliers configured for use with surgical clip cartridge assembly 300, reference may be made to China PCT Patent Application Serial No. PCT/CN2015/070733, filed on January 15, 2015, (Atty. Docket: 355677 (203-10414).

Also in accordance with the present disclosure, it is further contemplated that a surgical kit may be provided including a surgical clip applier 100 and a plurality of clip cartridge assemblies 300 including at least a first set of clip cartridge assemblies loaded with a stack of surgical clips having a first size and a second set of clip cartridge assemblies loaded with a stack of surgical clips having a second size different than the first size. The kit may include instructions for the assembly or surgical clip applier 100, the use of surgical clip applier 100, and the processing of surgical clip applier assembly 100 following use, a surgical clip applier 100 including a single handle assembly 102 and a single clip cartridge assembly 300, and a package, container or box configured to retain the same.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

## Claims

1. A clip cartridge assembly (300), comprising:
a clip tray (302) configured to be selectively loadable in a channel assembly (108) of a surgical clip applier (100), the clip tray (302) including a first plurality of distally oriented, deflectable, resilient fingers (302d) projecting from a base wall (302a) thereof;
a cartridge clip pusher bar (304) disposed adjacent the clip tray (302) and slidable relative thereto, the cartridge clip pusher bar (304) including a second plurality of distally oriented, deflectable, resilient fingers (304e) projecting from a base wall thereof and each terminating in a distal shoulder (304f) and a pusher defining a pair of spaced apart prongs (304d); and
a plurality of surgical clips ("C") interposed between the clip tray (302) and the cartridge clip pusher bar (304), wherein a surgical clip ("C") is disposed distally of each resilient finger (302d) of the clip tray (302),
wherein the distal-most finger (302d) of the first plurality of distally oriented, deflectable, resilient fingers (302d) has a rectangular configuration configured to engage a backspan or crown of the distal-most clip and nest between the spaced apart prongs;
and wherein each remaining finger of the first plurality of distally oriented, deflectable, resilient fingers (302d) includes a V-shaped notch formed in a distal end thereof.

2. The clip cartridge assembly (300) according to claim 1, wherein the cartridge clip pusher bar (304) further includes a stem (304c) disposed at and projecting from a proximal end (304a) of the cartridge clip pusher bar (304), the stem (304c) configured to slidably extend through a channel (306) defined in an inner surface of the clip tray (302), the cartridge clip pusher bar (304) being urged distally relative to the clip tray (302) by contact with the stem (304c) of the cartridge clip pusher bar (304).

3. The clip cartridge assembly (300) according to claim 1 or claim 2, wherein distal movement of the cartridge clip pusher bar (304) causes each distal shoulder (304f) of the cartridge clip pusher bar (304) to contact a backspan of a respective surgical clip ("C"), thereby distally advancing all of the surgical clips simultaneously.

4. The clip cartridge assembly (300) according to claim 2 or claim 3, wherein following distal movement of the cartridge clip pusher bar (304), upon a proximal movement of the cartridge clip pusher bar (304), each of the second plurality of distally oriented, deflectable, resilient fingers (304e) contacts the backspan of a respective remaining one of the surgical clip ("C") to proximally move all of the remaining surgical clips until the backspans of all of the remaining surgical clips contacts a respective finger of the first plurality of distally oriented, deflectable, resilient fingers (302d) of the clip tray (302) to block any further proximal movement of the remaining surgical clips ("C").

5. The clip cartridge assembly (300) according to any of claims 1 to 4, wherein the clip tray (302) further includes a spaced apart pair of distally oriented, deflectable, resilient fingers (302e) projecting from a distal end of the base wall (302a) thereof, the spaced apart pair of fingers (302e) configured to contact a distal surface of a backspan of a surgical clip ("C") being distally urged by the cartridge clip pusher bar (304), thereby inhibiting any further distal movement of the surgical clip ("C").

6. The clip cartridge assembly (300) according to any preceding claim, further including a channel cover (308) configured to selectively engage the clip tray (302), the channel cover (308) further configured to provide a biasing force on the cartridge clip pusher bar (304) such that the cartridge clip pusher bar (304) is maintained in an position adjacent to the clip tray (302).

7. A surgical clip applier (100), comprising:
a housing (104);
at least one handle (106) pivotably connected to the housing (104);
a channel assembly (108) extending distally from the housing (104);
a clip cartridge assembly (300) according to any of claims 1 to 6, selectively loadable in the channel assembly (108), the clip cartridge assembly (300) being in mechanical communication with the at least one handle (106),
wherein a pair of jaws (120) is supported on a distal end of the channel assembly (108), the pair of jaws configured to receive a single, unformed surgical clip ("C") therein.

8. The surgical clip applier according to claim 7, further including an endoscopic assembly (500) selectively connectable to the housing (104), wherein the channel assembly (108) is disposed within the endoscopic assembly (500).

9. The surgical clip applier according to claim 8 wherein the pair of jaws (120) are supported on a distal end of the endoscopic assembly (500).

## Patentansprüche

1. Klammer-Patronenanordnung (300), umfassend:
eine Klammerschale (302), die dafür konfiguriert ist, selektiv in eine Kanalanordnung (108) eines chirurgischen Klammerapplikators (100) geladen werden zu können, wobei die Klammerschale (302) eine erste Vielzahl distal ausgerichteter, biegsamer, elastischer Finger (302d) umfasst, die von einer Basiswand (302a) derselben vorstehen;
eine Patronenklammer-Schieberstange (304), die angrenzend an die Klammerschale (302) angeordnet und relativ dazu verschiebbar ist, wobei die Patronenklammer-Schieberstange (304) eine zweite Vielzahl distal ausgerichteter, biegsamer, elastischer Finger (304e), die von einer Basiswand davon vorstehen und jeweils in einer distalen Schulter (304f) enden, und einen Schieber, der ein Paar beabstandeter Zinken (304d) definiert, aufweist; und
mehrere chirurgische Klammern ("C"), die zwischen der Klammerschale (302) und der Patronenklammer-Schieberstange (304) angeordnet sind, wobei eine chirurgische Klammer (_{"}C") distal von jedem elastischen Finger (302d) der Klammerschale (302) angeordnet ist, wobei der am weitesten distal liegende Finger (302d) der ersten Mehrzahl von distal ausgerichteten, biegsamen, elastischen Fingern (302d) eine rechteckige Konfiguration aufweist, die dafür konfiguriert ist, mit einer Rückfläche oder einer Krone der am weitesten distal liegenden Klammer und der Aufnahme zwischen den beabstandeten Zinken in Eingriff zu kommen;
und wobei jeder verbleibende Finger der ersten Vielzahl von distal ausgerichteten, biegsamen, elastischen Fingern (302d) eine V-förmige Kerbe aufweist, die in einem distalen Ende davon ausgebildet ist.

2. Klammer-Patronenanordnung (300) nach Anspruch 1, wobei die Patronenklammer-Schieberstange (304) ferner einen Schaft (304c) aufweist, der an einem proximalen Ende (304a) der Patronenklammer-Schieberstange (304) angeordnet ist und von diesem vorsteht, wobei der Schaft (304c) so konfiguriert ist, dass er sich gleitend durch einen Kanal (306) erstreckt, der in einer Innenfläche der Klammerschale (302) definiert ist, wobei die Patronenklammer-Schieberstange (304) durch Kontakt mit dem Schaft (304c) der Patronenklammer-Schieberstange (304) distal relativ zu der Klammerschale (302) gedrückt wird.

3. Klammer-Patronenanordnung (300) nach Anspruch 1 oder Anspruch 2, wobei eine distale Bewegung der Patronenklammer-Schieberstange (304) bewirkt, dass jede distale Schulter (304f) der Patronenklammer-Schieberstange (304) mit einer Rückfläche einer entsprechenden chirurgischen Klammer (_{"}C") in Kontakt kommt, wodurch alle chirurgischen Klammern gleichzeitig distal vorgeschoben werden.

4. Klammer-Patronenanordnung (300) nach Anspruch 2 oder Anspruch 3, wobei nach einer distalen Bewegung der Patronenklammer-Schieberstange (304) jede der zweiten Vielzahl von distal ausgerichteten, biegsamen elastischen Fingern (304e) die Rückfläche einer entsprechenden verbleibenden chirurgischen Klammer (_{"}C") nach einer proximalen Bewegung der Patronenklammer-Schieberstange (304) berührt, um alle verbleibenden chirurgischen Klammern proximal zu bewegen, bis die Rückflächen aller verbleibenden chirurgischen Klammern einen entsprechenden Finger der ersten Vielzahl von distal ausgerichteten, biegsamen, elastischen Fingern (302d) der Klammerschale (302) berühren, um jede weitere proximale Bewegung der verbleibenden chirurgischen Klammern (_{"}C") zu blockieren.

5. Klammer-Patronenanordnung (300) nach einem der Ansprüche 1 bis 4, wobei die Klammerschale (302) ferner ein beabstandetes Paar distal ausgerichteter, biegsamer, elastischer Finger (302e) aufweist, die von einem distalen Ende der Basiswand (302a) derselben vorstehen, wobei das beabstandete Paar Finger (302e) so konfiguriert ist, dass es eine distale Oberfläche einer Rückfläche einer chirurgischen Klammer ("C") berührt, die durch die Patronenklammer-Schieberstange (304) distal gedrückt wird, wodurch jede weitere distale Bewegung der chirurgischen Klammer ("C") verhindert wird.

6. Klammer-Patronenanordnung (300) nach einem der vorhergehenden Ansprüche, ferner umfassend eine Kanalabdeckung (308), die so konfiguriert ist, dass sie selektiv in die Klammerschale (302) eingreift, wobei die Kanalabdeckung (308) ferner so konfiguriert ist, dass sie eine Vorspannkraft auf die Patronenklammer-Schieberstange (304) ausübt, so dass die Patronenklammer-Schieberstange (304) in einer Position neben der Klammerschale (302) gehalten wird.

7. Chirurgischer Klammer-Applikator (100), umfassend:
ein Gehäuse (104);
mindestens einen Griff (106), der schwenkbar mit dem Gehäuse (104) verbunden ist;
eine Kanalanordnung (108), die sich distal vom Gehäuse (104) erstreckt;
eine Klammer-Patronenanordnung (300) nach einem der Ansprüche 1 bis 6, die selektiv in die Kanalanordnung (108) geladen werden kann, wobei die Klammer-Patronenanordnung (300) in mechanischer Verbindung mit dem mindestens einen Griff (106) steht, wobei ein Backenpaar (120) an einem distalen Ende der Kanalanordnung (108) gelagert ist, wobei das Backenpaar dafür konfiguriert ist, eine einzelne, nicht geformte chirurgische Klammer (_{"}C") darin aufzunehmen.

8. Chirurgischer Klammer-Applikator nach Anspruch 7, ferner umfassend eine endoskopische Anordnung (500), die selektiv mit dem Gehäuse (104) verbindbar ist, wobei die Kanalanordnung (108) innerhalb der endoskopischen Anordnung (500) angeordnet ist.

9. Chirurgischer Klammer-Applikator nach Anspruch 8, wobei das Backenpaar (120) an einem distalen Ende der endoskopischen Anordnung (500) gelagert ist.

## Revendications

1. Ensemble cartouche d'agrafes (300), comprenant :
un plateau d'agrafes (302) conçu pour être sélectivement chargeable dans un ensemble canal (108) d'un applicateur d'agrafes chirurgicales (100), le plateau d'agrafes (302) comportant une première pluralité de doigts (302d) élastiques orientés distalement et déformables faisant saillie à partir d'une de ses parois de base (302a) ;
une barre de poussée d'agrafe de cartouche (304) disposée adjacente au plateau d'agrafes (302) et pouvant coulisser par rapport à celui-ci, la barre de poussée d'agrafe de cartouche (304) comportant une seconde (304e) pluralité de doigts élastiques orientés distalement et déformables, faisant saillie à partir d'une de ses parois de base et chacun se terminant par un épaulement distal (304f) et un poussoir définissant une paire de dents espacées(304d) ; et
une pluralité d'agrafes chirurgicales (« C ») interposées entre le plateau d'agrafes (302) et la barre de poussée d'agrafe de cartouche (304), une agrafe chirurgicale (« C ») étant disposée distalement par rapport à chaque doigt (302d) élastique du plateau d'agrafes (302), le doigt (302d) le plus distal de la première pluralité de doigts (302d) élastiques orientés distalement et déformables ayant une configuration rectangulaire conçue pour venir en prise avec une barre transversale ou le sommet de l'agrafe la plus distale et pour s'emboîter entre les dents espacées ;
et chaque doigt restant de la première pluralité de doigts (302d) élastiques orientés distalement et déformables comportant une encoche en forme de V formée dans une de ses extrémités distales.

2. Ensemble cartouche d'agrafes (300) selon la revendication 1, dans lequel la barre de poussée d'agrafe de cartouche (304) comporte en outre une tige (304c) disposée au niveau d'une extrémité proximale (304a) de la barre de poussée d'agrafe de cartouche (304) et faisant saillie à partir de celle-ci, la tige (304c) étant conçue pour s'étendre de manière coulissante à travers un canal (306) défini dans une surface intérieure du plateau d'agrafes (302), la barre de poussée d'agrafe de cartouche (304) étant sollicitée distalement par rapport au plateau d'agrafes (302) par contact avec la tige (304c) de la barre de poussée d'agrafe de cartouche (304).

3. Ensemble cartouche d'agrafes (300) selon la revendication 1 ou la revendication 2, dans lequel le mouvement distal de la barre de poussée d'agrafe de cartouche (304) amène chaque épaulement distal (304f) de la barre de poussée d'agrafe de cartouche (304) à entrer en contact avec une barre transversale d'une agrafe chirurgicale (« C ») respective, faisant ainsi avancer distalement toutes les agrafes chirurgicales simultanément.

4. Ensemble cartouche d'agrafes (300) selon la revendication 2 ou la revendication 3, dans lequel après un mouvement distal de la barre de poussée d'agrafe de cartouche (304), lors d'un mouvement proximal de la barre de poussée d'agrafe de cartouche (304), chacun de la seconde (304e) pluralité de doigts élastiques orientés distalement et déformables entre en contact avec la barre transversale d'une agrafe chirurgicale (« C ») restante respective pour déplacer de manière proximale toutes les agrafes chirurgicales restantes jusqu'à ce que les barres transversales de toutes les agrafes chirurgicales restantes soient en contact avec un doigt respectif de la première pluralité de doigts élastiques (302d) orientés distalement et déformables du plateau d'agrafes (302) pour bloquer tout autre mouvement proximal des agrafes chirurgicales (« C ») restantes.

5. Ensemble cartouche d'agrafes (300) selon l'une quelconque des revendications 1 à 4, dans lequel le plateau d'agrafes (302) comporte en outre une paire (302e) espacée de doigts élastiques orientés distalement et déformables faisant saillie depuis une extrémité distale de sa paroi de base (302a), la paire (302e) espacée de doigts étant conçue pour entrer en contact avec une surface distale d'une barre transversale d'une agrafe chirurgicale (« C ») étant sollicitée distalement par la barre de poussée de l'agrafe de cartouche (304), empêchant ainsi tout autre mouvement distal de l'agrafe chirurgicale (« C »).

6. Ensemble cartouche d'agrafes (300) selon l'une quelconque des revendications précédentes, comportant en outre un couvercle de canal (308) conçu pour venir en prise sélectivement avec le plateau d'agrafes (302), le couvercle de canal (308) étant en outre conçu pour fournir une force de sollicitation sur la barre de poussée d'agrafe de cartouche (304) de telle sorte que la barre de poussée d'agrafe de cartouche (304) est maintenue dans une position adjacente au plateau d'agrafes (302).

7. Applicateur d'agrafes chirurgicales (100), comprenant :
un boîtier (104) ;
au moins une poignée (106) reliée de manière pivotante au boîtier (104) ;
un ensemble canal (108) s'étendant distalement à partir du boîtier (104) ;
un ensemble cartouche d'agrafes (300) selon l'une quelconque des revendications 1 à 6, pouvant être chargé sélectivement dans l'ensemble canal (108), l'ensemble cartouche d'agrafes (300) étant en communication mécanique avec l'au moins une poignée (106), une paire des mâchoires (120) étant supportée sur une extrémité distale de l'ensemble canal (108), la paire de mâchoires étant conçue pour recevoir une seule agrafe chirurgicale (« C ») non formée à l'intérieur.

8. Applicateur d'agrafes chirurgicales selon la revendication 7, comportant en outre un ensemble endoscopique (500) pouvant être relié sélectivement au boîtier (104), l'ensemble canal (108) étant disposé à l'intérieur de l'ensemble endoscopique (500).

9. Applicateur d'agrafes chirurgicales selon la revendication 8, dans lequel la paire de mâchoires (120) est supportée sur une extrémité distale de l'ensemble endoscopique (500).
